Europäisches Patentamt

European Patent Office

Office européen des brevets

(11) Publication number: **0 210 845**
**B1**

# EUROPEAN PATENT SPECIFICATION

(45) Date of publication of patent specification: **28.06.89**

(51) Int. Cl.⁴: **G 01 N 33/24**

(21) Application number: **86305712.1**

(22) Date of filing: **24.07.86**

(54) Rock analyser.

(30) Priority: **25.07.85 GB 8518821**

(43) Date of publication of application:
**04.02.87 Bulletin 87/06**

(45) Publication of the grant of the patent:
**28.06.89 Bulletin 89/26**

(84) Designated Contracting States:
**BE DE FR GB IT NL**

(56) References cited:
**DE-A-2 701 782**
**FR-A-2 435 038**
**US-A-2 871 105**
**US-A-4 106 908**

(73) Proprietor: **The British Petroleum Company
p.l.c.
Britannic House Moor Lane
London EC2Y 9BU (GB)**

(72) Inventor: **Crisp, Russell Ian British Petroleum
Co. p.l.c.
Chertsey Road Sunbury-on-Thames
Middlesex TW16 7LN (GB)**

(74) Representative: **MacLeod, Malcolm et al
BP INTERNATIONAL LIMITED Patents Division
Chertsey Road
Sunbury-on-Thames Middlesex TW16 7LN (GB)**

Courier Press, Leamington Spa, England.

**Description**

This invention relates to a portable rock analyser.

Organic geochemists routinely measure the organic content of rocks by laboratory pyrolysis. This organic matter may be divided into two types, volatile material (oil) and polymeric, involatile material (kerogen). On heating, kerogen decomposes to give volatile components (oil and gas) and an inert residue. Thus rocks containing significant quantities of labile kerogen are considered to be hydrocarbon source rocks. Conventional laboratory procedure for identifying source rocks includes a two-stage pyrolysis in an inert atmosphere with the expelled hydrocarbons being detected by a flame ionization detector (FID). The first stage of the pyrolysis takes place between 250° and 300°C and is intended to detect only the oil. The second stage occurs at 500°—600°C, at which temperatures kerogen breakdown rapidly occurs. The two values obtained are referred to as S1 or P1 (low temperature products) and S2 or P2 (high temperature products). The units are usually kg (hydrocarbons) per tonne (rock). A typical laboratory determination takes 20—30 minutes and uses carefully prepared and finely ground rock samples.

In US—A—4 106 908 a laboratory apparatus requiring gas supplies, valves, a gas chromatography column and a pyrolyzing tube is disclosed. A heater is situated outside the pyrolysis tube into which the sample is placed.

Considerable effort is spent performing a great many of these analyses as a means of identifying rich samples, suitable for further geochemical analysis. The majority of samples tested contain little or no organic material, however.

It is an object of the present invention to provide a portable rock analyser for use in field screening tests to reduce the number of worthless samples sent to laboratories for analysis. This will, in turn, allow more expedient analyses of the richer samples.

Thus according to the present invention, there is provided a rock analyser comprising a portable combination of a chamber and a hydrocarbon detector, the chamber containing in the same environment a heater and the sensor of the hydrocarbon detector, the heater containing the sample and being connectable to a source of power and the sensor being connected to the remainder of the hydrocarbon detector situated outside the chamber.

Hydrocarbon detectors are commercially available. Suitable models include the GL-10 detector for methane manufactured by Molecular Controls Ltd of Leeds, England and the AG 5100 detector for methane, manufactured by International Sensor Technology of California, USA, and described in US patents 3,955,268 and 4,013,943. These are rechargeable battery powered detectors designed for atmospheric monitoring. Whilst the units are primarily designed to detect methane they have been shown to respond to most volatile hydrocarbons. Full Scale Deflection (FSD) corresponds to 5% methane by volume.

The heater may be a ceramic furnace incorporating a heating coil which may be wound from resistance wire and be connectable via a switch to a battery.

The heater may incorporate a timer for better reproducibility.

The heater is heated to a temperature of around 700°—1000°C in less than 30 seconds when the switch is operated. The temperature and rate of heating are such that an inert atmosphere is not necessary in the pyrolysis chamber to ensure degradation rather than combustion. The volume of the chamber should be such that pyrolysis of a 30 mg sample of rock with a combined hydrocarbon yield (P1+P2) of 50 kg tonne$^{-1}$ would give a theoretical concentration of 5% of hydrocarbons with carbon numbers less than 10 (i.e. full scale deflection). This volume is 100 cm$^3$. Thus the most sensitive range of the instrument is between 1 and 10 kg tonne$^{-1}$ for 30 mg samples.

In practice the range will depend on the actual proportion of the generated hydrocarbon sufficiently volatile to give a detector response, the distribution of these products (since the detector is most sensitive to the lighter hydrocarbons) and the size of the samples tested. The 30 mg sample size is chosen as being a convenient size to handle in the field, without requiring large amounts of power to pyrolyse it.

The instrument is operated by selecting a suitably sized piece of rock, inserting it into the heater and replacing the lid of the cylinder. The power button is depressed for 20—40 seconds and then released. After allowing a further 30—60 seconds for the instrument to stabilize, the detector reading is recorded. The lid is then removed, allowing clean air to purge the detector. The heater can be cleaned by simply depressing the power button for a few seconds. The instrument is then ready to analyse another sample. The whole process takes less than five minutes. Alternatively, the procedure can be automatically controlled by a timer.

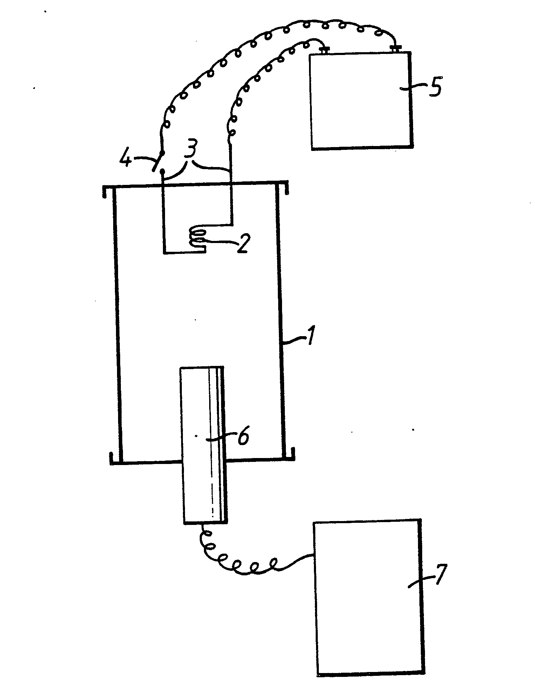

The invention is illustrated with reference to the accompanying drawing.

The analyser comprises a cylindrical chamber 1 of volume 100 cm$^3$ containing a heating coil 2 wound from four cm of resistance wire (80% Ni, 20% Cr) connected via electrodes 3 and a switch 4 to a two volt lead acid battery 5.

The chamber also contains the sensor 6 of a hydrocarbon detector sold by international Sensor Technology under the reference AG 5100. The sensor 6 is connected to the remainder of the detector 7 which is located outside the chamber 1.

By selecting a suitable cut-off point, below which samples are considered worthless, the instrument can be used to screen samples in the field prior to full geochemical analysis in the laboratory.
This is illustrated by the following examples:

Example 1

Pieces of rock of similar size and approximately 30 mg in weight were analysed using the portable instrument described above. A cut-off of 10% FSD was used. All samples were subsequently analysed in the laboratory to check the findings.

The results are presented in Table 1. Samples 1—9 were correctly identified as having little or no hydrocarbon potential. Samples 14—25 were correctly identified as having potentials greater than 1 kg tonne$^{-1}$. Samples 10—13 were identified as having significant potential but were later shown to have little or no potential.

Thus the instrument gives readings which err on the side of caution—in no case was a rich sample mis-identified as being poor. In a real field study this exercise would have saved over one third of the laboratory effort.

TABLE 1

| Sample No. | Portable instrument reading (% FSD) | Laboratory pyrolysis yield (kg tonne$^{-1}$) |
|---|---|---|
| 1 | 4 | 0.3 |
| 2 | 5 | 0.2 |
| 3 | 3 | 0.2 |
| 4 | 3 | 0.1 |
| 5 | 2 | 0 |
| 6 | 2 | 0 |
| 7 | 4 | 0.2 |
| 8 | 1 | 0 |
| 9 | 7 | 0.5 |
| 10 | 18 | 0.8 |
| 11 | 21 | 0.7 |
| 12 | 14 | 0.3 |
| 13 | 17 | 0.8 |
| 14 | 85 | 2.6 |
| 15 | 100 | 4.6 |
| 16 | 53 | 1.7 |
| 17 | 71 | 2.0 |
| 18 | 63 | 2.3 |
| 19 | 63 | 2.2 |
| 20 | 28 | 1.6 |
| 21 | greater than 100 | 10.2 |
| 22 | 65 | 1.9 |
| 23 | 100 | 4.4 |
| 24 | 53 | 1.8 |
| 25 | greater than 100 | 21.0 |

Example 2

A further series of tests were carried out in which the detector was fitted with a digital read-out expressed in kg hydrocarbon material per tonne of rock.

The results set out in Table 2 show good agreement between the instrument readings and subsequent analyses carried out under laboratory conditions.

TABLE 2

| Sample No. | Portable instrument reading (kg/tonne) | Laboratory pyrolysis yield (kg/tonne) |
|---|---|---|
| 26 | 30 | 28.0 |
| 27 | 6 | 3.5 |
| 28 | 0.5 | 0.4 |
| 29 | 1.5 | 2.1 |
| 30 | 35 | 17 |
| 31 | 0 | 0 |
| 32 | 2 | 3.3 |
| 33 | 4.5 | 5.8 |
| 34 | 20 | 13.8 |
| 35 | 5 | 8.5 |
| 36 | 16 | 15.8 |
| 37 | 33 | 23.4 |
| 38 | 2 | 2.6 |
| 39 | greater than 50 | 109 |
| 40 | 2 | 2.6 |
| 41 | 34 | 19.4 |
| 42 | 2 | 1.7 |
| 43 | 0.5 | 0 |
| 44 | 0.4 | 0 |
| 45 | greater than 50 | 90.2 |
| 46 | 1 | 1.6 |
| 47 | 1.5 | 0.6 |
| 48 | 50 | 68.3 |
| 49 | greater than 50 | 97.4 |
| 50 | 0.6 | 0.2 |
| 51 | 8 | 9.5 |
| 52 | 35 | 28 |
| 33 | 46 | 43.6 |
| 54 | 38 | 48 |
| 55 | 31 | 18 |
| 56 | 1.5 | 2 |
| 57 | 2 | 2 |

## Claims

1. A rock analyser comprising a portable combination of a chamber (1) and a hydrocarbon detector (7), the chamber containing in the same environment a heater (2) and the sensor (6) of the hydrocarbon detector, the heater containing the sample and being connectable to a source of power (5), and the sensor being connected to the remainder of hhe hydrocarbon detector situated outside the chamber.

2. A rock analyser according to claim 1 wherein the heater is a ceramic furnace incorporating a heating coil.

3. A rock analyser according to claim 2 wherein the heating coil is fabricated from electrical resistance wire and the source of power is an electric battery.

4. A rock analyser according to any of the preceding claims wherein the hydrocarbon detector is a methane detector.

## Patentansprüche

1. Gesteins-Analysengerät, umfassend eine tragbare Kombination aus einer Kammer (1) und einem Kohlenwasserstoff-Detektor (7), wobei die Kammer in der gleichen Umgebung eine Heizquelle (2) und den Sensor (6) des Kohlenwasserstoff-Detektors enthält, wobei die Heizquelle die Probe enthält und sich mit einer Spannungsquelle (5) verbinden läßt, und wobei der Sensor mit dem außerhalb der Kammer befindlichen Rest des Kohlenwasserstoff-Detektors verbunden ist.

2. Gesteins-Analysengerät nach Anspruch 1, worin die Heizquelle ein eine eingebaute Heizwicklung enthaltender keramischer Ofen ist.

3. Gesteins-Analysengerät nach Anspruch 2, worin die Heizwicklung aus elektrischem Widerstandsdraht gefertigt ist und die Spannungsquelle eine elektrische Batterie ist.

4. Gesteins-Analysengerät nach irgendeinem der vorhergehenden Ansprüche, worin der Kohlenwasserstoff-Detektor ein Methan-Detektor ist.

# EP 0 210 845 B1

**Revendications**

1. Analyseur de roches comprenant une combinaison portative d'une chambre (1) et d'un détecteur (7) d'hydrocarbures, la chambre contenant, dans la même enceinte, un dispositif de chauffage (2) et le capteur (6) du détecteur d'hydrocarbures, le dispositif de chauffage contenant l'échantillon et étant destiné à être connecté à une source d'énergie (5), et le capteur étant connecté au reste du détecteur d'hydrocarbures placé à l'extérieur de la chambre.

2. Analyseur de roches selon la revendication 1, dans lequel le dispositif de chauffage est un four céramique contenant un enroulement de chauffage.

3. Analyseur de roches selon la revendication 2, dans lequel l'enroulement de chauffage est formé d'un fil électrique de résistance et la source d'énergie est une batterie d'accumulateurs électriques.

4. Analyseur de roches selon l'une quelconque des revendications précédentes, dans lequel le détecteur d'hydrocarbures est un détecteur de méthane.